# EUROPEAN PATENT APPLICATION

(11) **EP 2 213 234 A1**
(43) Date of publication of application: **04.08.2010**
(21) Application number: 10151663.1
(22) Date of filing: 26.01.2010
(51) Int. Cl.: A61B 6/02, G06T 11/00

(54) **Radiographic tomosynthesis image generating apparatus**

(30) Priority: 28.01.2009 JP 2009016707; 27.11.2009 JP 2009270553
(71) Applicant: FUJIFILM Corporation, Minato-ku Tokyo 106-8620 (JP)
(72) Inventor: Akahori, Sadato, Kanagawa 258-8538 (JP); Enomoto, Jun, Kanagawa 258-8538 (JP); Sawada, Hirofumi, Kanagawa 258-8538 (JP); Kanagawa, Eiichi, Kanagawa 258-8538 (JP); Nishimura, Tomoyoshi, Kanagawa 258-8538 (JP); Ohta, Yasunori, Kanagawa 258-8538 (JP); Ida, Noriaki, Kanagawa 258-8538 (JP)
(74) Representative: Höhfeld, Jochen

(57) **Abstract**

A radiographic tomography image generating apparatus includes a radiographic image acquiring assembly (14) for moving a radiation applicator (20), which is disposed in confronting relation to a radiation detecting device (12), successively to a plurality of positions, controlling the radiation applicator (20) to apply radiation (26) to a subject (24) disposed over the radiation detecting device (12) in different directions while the radiation applicator (20) moves successively to the plurality of positions, and storing a plurality of radiographic images output successively from the radiation detecting device (12) in an image memory (30). The radiographic tomography image generating apparatus also includes an image reconstructor (16) for processing the radiographic images stored in the image memory (30) in order to reconstruct a diagnostic tomography image of the subject (24) according to a reconstructing process, and a preview display unit (100) for displaying preview images on a display monitor (17) based on the radiographic images output successively from the radiation detecting device (12).

## Description

### BACKGROUND OF THE INVENTION

### Field of the Invention:

The present invention relates to a radiographic tomography image generating apparatus for capturing a tomography image utilizing a radiographic image capturing process.

### Description of the Related Art:

In recent years, in order for X-ray image capturing apparatus to conduct detailed observations of a local region of a subject, there has been proposed a tomosynthesis image capturing process, in which an X-ray tube is moved over the subject while applying X-rays from the X-ray tube toward the subject at different angles to thereby capture radiographic images of the subject. The captured radiographic images are added together to generate a tomography image, which represents, with emphasis, a desired sectional plane across the subject (see, for example, Japanese Laid-Open Patent Publication No. 57-203430).

In a tomosynthesis image capturing process, an X-ray tube is moved either parallel to a detector such as a flat panel or the like, or along an arcuate track such as a circular track or an elliptical track, in order to capture a plurality of radiographic images of the subject at different angles. The captured radiographic images are processed to reconstruct a tomographic image of the subject. The tomographic image can be generated by translating the radiographic images, or by adjusting the sizes of the radiographic images and adding the adjusted radiographic images (see, for example, Japanese Laid-Open Patent Publication No. 2008-043757).

There has recently been proposed a radiographic tomography image generating apparatus for generating a simple tomography image to specify a sectional plane by means of a simple reconstructing process (see, for example, Japanese Laid-Open Patent Publication No. 2008-154647). The proposed radiographic tomography image generating apparatus includes a detecting means for detecting transmissive images of an examinee in different directions in order to produce a plurality of transmissive image data, a first synthesizing means for synthesizing relatively small amounts of information from the plurality of transmissive image data in order to generate three-dimensional image data of the examinee having low resolution, a specifying input means for accepting an input for specifying a desired sectional plane from the user in a simple three-dimensional image, which is represented by the three-dimensional image data having low resolution, and a second synthesizing means for synthesizing relatively large amounts of information of the plurality of transmissive image data in order to generate sectional-plane image data having high resolution in the specified sectional plane.

As disclosed in Japanese Laid-Open Patent Publication No. 57-203430 and Japanese Laid-Open Patent Publication No. 2008-043757, however, since the radiographic tomography image generating apparatus, which is capable of reconstructing a desired tomography image, captures a plurality of images of a subject based on a single image capture start instruction, the image capturing time takes several seconds from the start of the image capturing process to the end of the image capturing process, and the subject may possibly move during the period in which image capturing takes place.

If motion of the subject (body motion) during the time in which the image is captured is of a level too small to adversely affect reconstruction of the tomography image, then no significant problems will arise. However, if the level of body motion of the subject is large enough to adversely affect reconstruction of the tomography image, then any images captured subsequently to such body motion will be wasted, and another image capturing process will be required. Consequently, the radiographic tomography image generating apparatus is potentially low in efficiency, may cause the subject to be exposed to an excessively high dosage of radiation, and may hold the subject constrained for an unduly long period of time.

The above problems also hold true for the radiographic tomography image generating apparatus disclosed in Japanese Laid-Open Patent Publication No. 2008-154647. The radiographic tomography image generating apparatus disclosed therein operates as follows: After a series of radiographic images of an examinee have been captured, the first synthesizing means generates three-dimensional image data of the examinee having low resolution from radiographic images that are stored in an image memory. Then, the operator specifies a desired sectional plane with the specifying input means, while referring to a display image represented by the three-dimensional image data of low resolution. Thereafter, the second synthesizing means generates sectional-plane image data having high resolution in the specified sectional plane from the stored radiographic images. Since the first synthesizing means and the second synthesizing means are unable to begin processing sequences unless the series of radiographic images of the examinee have been captured, the radiographic tomography image generating apparatus fails to recognize body motions of the examinee in real time while the radiographic images of the examinee are being captured.

### SUMMARY OF THE INVENTION

It is an object of the present invention to provide a radiographic tomography image generating apparatus, which includes a display device for displaying captured radiographic images of a subject during a radiographic image capturing process, in order to enable an operator to easily recognize the level of body motion of the subject during the radiographic image capturing process, so that the radiographic tomography image generating apparatus can operate efficiently. Accordingly, the radiographic tomography image generating apparatus does not cause the subject to be exposed to an excessively high dosage of radiation, and does not require the subject to be constrained for an unduly long period of time.

According to the present invention, a radiographic tomography image generating apparatus includes a radiation detecting device, a radiation applicator disposed in confronting relation to the radiation detecting device, a radiographic image acquiring assembly for moving the radiation applicator successively to a plurality of positions, controlling the radiation applicator to apply radiation to a subject disposed over the radiation detecting device in different directions while the radiation applicator moves successively to the positions, and storing a plurality of radiographic images output successively from the radiation detecting device in an image memory. The radiographic tomography image generating apparatus also includes an image reconstructor for processing the radiographic images stored in the image memory to reconstruct a diagnostic tomography image of the subject according to a reconstructing process, and a preview display unit for displaying preview images based on the radiographic images output successively from the radiation detecting device.

With the above arrangement, captured radiographic images of the subject can be displayed as they are being captured, thus enabling the operator of the radiographic tomography image generating apparatus to easily recognize the level of body motion of the subject. Therefore, the radiographic tomography image generating apparatus can operate efficiently, does not cause the subject to be exposed to an excessively high dosage of radiation, and does not require the subject to be constrained for an unduly long period of time.

The preview display unit may display the preview images by displaying radiographic images at a lower resolution than the radiographic images that are output successively from the radiation detecting device.

The preview display unit may display the preview images by displaying radiographic images of a particular region or a specified region.

The preview display unit may display the preview images by sequentially displaying at least every pth (p = 1, 2, 3, ···) output radiographic image.

The preview display unit may display the preview images by displaying radiographic images along with a reference pattern.

The radiographic tomography image generating apparatus may further include a preview processor for generating a preview tomography image by processing radiographic images that are output successively from the radiation detecting device, according to a simpler reconstructing process than the reconstructing process carried out by the image reconstructor, in order that the preview display unit may display the preview tomography image.

With the above arrangement, a preview tomography image based on radiographic images of the subject while the radiographic images are being captured can be displayed, thus enabling the operator to easily recognize the level of body motion of the subject. Therefore, the radiographic tomography image generating apparatus can operate efficiently, does not cause the subject to be exposed to an excessively high dosage of radiation, and does not require the subject to be constrained for an unduly long period of time.

The preview processor may generate the preview tomography image at a lower resolution than the diagnostic tomography image reconstructed by the image reconstructor.

The image reconstructor may generate the diagnostic tomography image, which represents a plurality of regions in the subject, and the preview processor may generate the preview tomography image, which represents a particular region or a specified region among the plurality of regions.

The preview processor may update the preview tomography image by processing at least a given every qth (q = 1, 2, 3, ···) radiographic image output from the radiation detecting device by way of backprojection, whereas the preview display unit may update the displayed preview tomography image each time the preview processor updates the preview tomography image.

The preview processor may control the preview display unit to display the preview tomography image along with a reference pattern.

The image reconstructor may generate diagnostic tomography images, which represent at least two sectional planes in the subject, whereas the preview processor may generate the preview tomography image, which represents a particular one of the at least two sectional planes.

The image reconstructor may generate the diagnostic tomography image by way of filtered backprojection, and the preview processor may generate the preview tomography image by way of simple backprojection.

The image reconstructor may generate the diagnostic tomography image while also using the preview tomography image generated by the preview processor.

The preview processor may generate the preview tomography image by processing every mth (m = 1, 2, 3, ···) radiographic image of the radiographic images that are output successively from the radiation detecting device, and the image reconstructor may generate the diagnostic tomography image using the generated preview tomography image and radiographic images that are not used by the preview processor.

The preview processor may generate every nth (n = 1, 2, 3, ···) preview tomography image by processing every mth (m = 1, 2, 3, ···) radiographic image of the radiographic images that are output successively from the radiation detecting device, and the image reconstructor may generate the diagnostic tomography image using the generated preview tomography images and radiographic images that are not used by the preview processor with respect to sectional planes in the subject for which the diagnostic tomography image is generated, and the image reconstructor may generate the diagnostic tomography image using radiographic images with respect to sectional planes in the subject for which the diagnostic tomography image is not generated.

The preview processor may generate the preview tomography image based on the radiographic images stored in the image memory.

The preview processor may generate the preview tomography image based on radiographic images that are output from the radiation detecting device, before the radiographic images are stored in the image memory.

According to the present invention, the radiographic tomography image generating apparatus, which is arranged as described above, can display captured radiographic images of the subject while they are being captured, thus enabling the operator of the radiographic tomography image generating apparatus to easily recognize the level of body motion of the subject. Therefore, the radiographic tomography image generating apparatus can operate efficiently, does not expose the subject to an excessively high dosage of radiation, and does not require the subject to be constrained for an unduly long period of time.

The above and other objects, features, and advantages of the present invention will become more apparent from the following description when taken in conjunction with the accompanying drawings in which preferred embodiments of the present invention are shown by way of illustrative example.

### BRIEF DESCRIPTION OF THE DRAWINGS

FIG. 1 is a schematic view, partially in block form, of a first image generating apparatus, which makes up a radiographic tomography image generating apparatus according to a first embodiment of the present invention;
FIG. 2A is a schematic view showing the manner in which a radiation applicator and a radiation detecting device, disposed on respective sides of a subject, move synchronously in mutually opposite horizontal directions in the first image generating apparatus;
FIG. 2B is a schematic view showing the manner in which a radiation applicator disposed on one side of a subject moves along an arcuate track with respect to a radiation detecting device disposed on the other side of the subject in the first image generating apparatus;
FIG. 3 is a circuit diagram, partially in block form, of a radiation detector incorporated in the radiation detecting device of the first image generating apparatus;
FIG. 4 is a schematic view, partially in block form, of a second image generating apparatus, which makes up a radiographic tomography image generating apparatus according to a second embodiment of the present invention;
FIGS. 5A through 5G are schematic views illustrating first through seventh radiographic images, respectively, which are produced by imaging first through third regions of interest of a subject from respective first through seventh positions, in a radiographic image capturing process performed by the second image generating apparatus;
FIGS. 6A through 6G are schematic views illustrating a process of emphasizing a second region of interest by shifting and adding first through third radiographic images and fifth through seventh radiographic images with respect to fourth radiographic images, which are used as a reference;
FIG. 7 is a view showing a subject according to another example;
FIG. 8 is a schematic view, partially in block form, of a second image generating apparatus according to a modification of the present invention;
FIG. 9 is a schematic view, partially in block form, of a third image generating apparatus, which makes up a radiographic tomography image generating apparatus according to a third embodiment of the present invention;
FIG. 10A is a block diagram illustrating a preview image reconstructing process, based on a first processing method carried out by the third image generating apparatus;
FIG. 10B is a block diagram illustrating a diagnostic image reconstructing process, based on the first processing method carried out by the third image generating apparatus;
FIG. 11A is a block diagram illustrating a preview image reconstructing process, based on a second processing method carried out by the third image generating apparatus;
FIG. 11B is a block diagram illustrating a diagnostic image reconstructing process, based on the second processing method carried out by the third image generating apparatus; and
FIG. 12 is a schematic view, partially in block form, of a third image generating apparatus according to a modification of the present invention.

### DESCRIPTION OF THE PREFERRED EMBODIMENTS

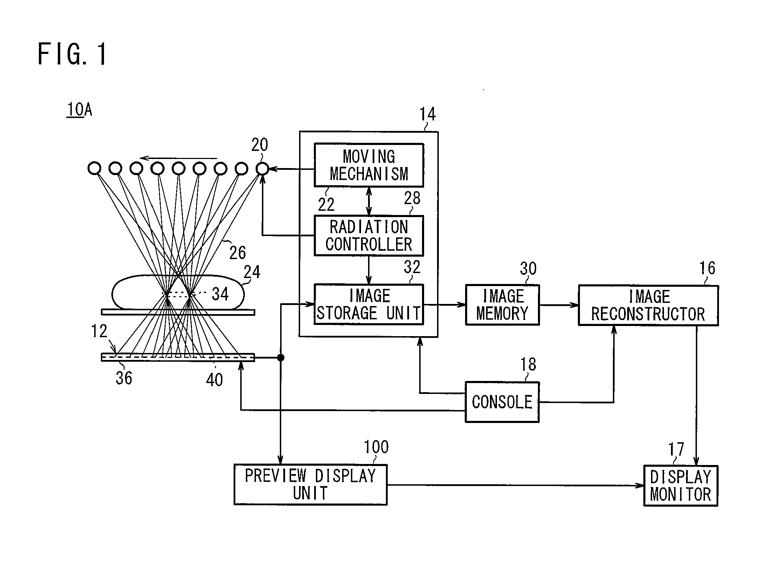

Radiographic tomography image generating apparatus (hereinafter referred to as "image generating apparatus") according to preferred embodiments of the present invention will be described below with reference to FIGS. 1 through 12. Like or corresponding parts are denoted by like or corresponding reference characters throughout the views.

As shown in FIG. 1, an image generating apparatus according to a first embodiment of the present invention (hereinafter referred to as a "first image generating apparatus 10A") includes a radiation detecting device 12, a radiographic image acquiring assembly 14, an image reconstructor 16, and a console (controller) 18 for controlling the radiation detecting device 12, the radiographic image acquiring assembly 14, and the image reconstructor 16.

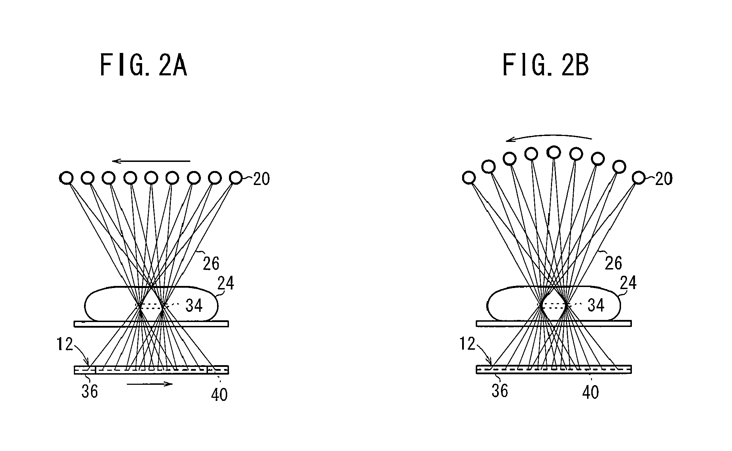

The radiographic image acquiring assembly 14 includes a radiation applicator 20 disposed in confronting relation to the radiation detecting device 12, a moving mechanism 22 for moving the radiation applicator 20 successively to a plurality of preset positions, a radiation controller 28 for controlling the radiation applicator 20 to apply radiation 26 to a subject 24 over the radiation detecting device 12 when the radiation applicator 20 reaches each of the preset positions, and an image storage unit 32 for storing radiographic images successively sent from the radiation detecting device 12 in chronological order, for example. The radiographic image acquiring assembly 14 operates to acquire a plurality of radiographic images from the radiation detecting device 12, when the radiation applicator 20 applies radiation 26 to the subject 24 over the radiation detecting device 12 in different directions, and while the radiation applicator 20 disposed in confronting relation to the radiation detecting device 12 moves successively to the preset positions. In the embodiment shown in FIG. 1, the moving mechanism 22 moves the radiation applicator 20 along a straight track while the radiation detecting device 12 is fixed in position. In FIG. 2A, the radiation applicator 20 and the radiation detecting device 12, which are disposed on respective sides of the subject 24, move synchronously in mutually opposite horizontal directions. In FIG. 2B, the radiation applicator 20 moves along an arcuate track while the radiation detecting device 12 remains fixed in position.

The radiographic image acquiring assembly 14 operates in two modes, i.e., a mode for capturing an individual radiographic image when the radiation applicator 20 reaches each of the preset positions, and a mode for capturing an image as a combination of individual radiographic images captured when the radiation applicator 20 reaches the respective preset positions. The former mode hereinafter will be referred to as a "radiographic image capturing mode" and the latter mode as a "tomosynthesis image capturing mode".

The image reconstructor 16 processes the radiographic images stored in the image memory 30 in order to generate or reconstruct a tomography image of the subject 24, i.e., a tomography image (diagnostic tomography image) of a region 34 of interest of the subject 24 who lies parallel to a detecting surface of the radiation detecting device 12, and displays the tomography image of the subject 24 on a display monitor 17. The image reconstructor 16 may reconstruct a tomography image according to a reconstructing process such as a simple backprojection process, or a filtered backprojection process, for example. The simple backprojection process is a process for backprojecting a plurality of radiographic images without applying a reconstruction filter, and then adding the plural radiographic images into a reconstructed image. There are two types of filtered backprojection processes. The first type is a process for applying a reconstruction filter as a convolution filter to a plurality of radiographic images, backprojecting the radiographic images, and then adding them into a reconstructed image. The second type is a process for Fourier-transforming a plurality of radiographic images into frequency-domain data, applying a reconstruction filter to the frequency-domain data, backprojecting the frequency-domain data, and thereafter adding them into a reconstructed image. Either of these filtered backprojection processes may be employed. The simple backprojection process and the filtered backprojection process will collectively be referred to as a "backprojection process".

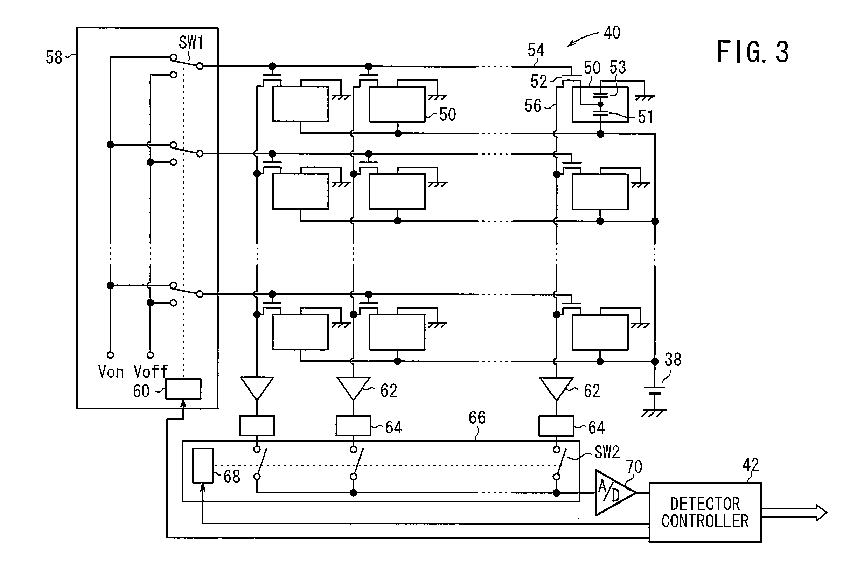

The radiation detecting device 12 includes a casing 36, a battery 38 (see FIG. 3) housed in the casing 36, a radiation detector 40 housed in the casing 36, and a detector controller 42 housed in the casing 36.

As shown in FIG. 3, the radiation detector 40 comprises an array of thin-film transistors (TFTs) 52 arranged in rows and columns, a photoelectric conversion layer 51 made of a material such as amorphous selenium (a-Se) for generating electric charges upon detection of radiation 26, the photoelectric conversion layer 51 being disposed on the array of TFTs 52, and an array of storage capacitors 53 connected to the photoelectric conversion layer 51. When radiation 26 is applied to the radiation detector 40, the photoelectric conversion layer 51 generates electric charges, and the storage capacitors 53 store the generated electric charges. Then, the TFTs 52 are turned on one row at a time to read the electric charges from the storage capacitors 53 as image signals. In FIG. 3, the photoelectric conversion layer 51 and one of the storage capacitors 53 are shown as forming a pixel 50, with the pixel 50 being connected to one of the TFTs 52. Details of the other pixels 50 are omitted from illustration. Since amorphous selenium tends to change in structure and lose functionality at high temperatures, amorphous selenium needs to be used within a certain temperature range. Therefore, some means for cooling the radiation detector 40 preferably should be provided in the casing 36.

The TFTs 52 connected to the respective pixels 50 are connected to respective gate lines 54 that extend parallel to the rows, and to respective signal lines 56 that extend parallel to the columns. The gate lines 54 are connected to a line scanning driver 58, and the signal lines 56 are connected to a multiplexer 66, which serves as a reading circuit.

The gate lines 54 are supplied with control signals Von, Voff for turning on and off the TFTs 52 along the rows from the line scanning driver 58. The line scanning driver 58 comprises a plurality of first switches SW1 for switching between the gate lines 54, and an address decoder 60 for outputting a selection signal for selecting one of the first switches SW1 at a time. The address decoder 60 is supplied with an address signal from the detector controller 42.

The signal lines 56 are supplied with electric charges stored in the storage capacitors 53 of the pixels 50 through the TFTs 52 arranged in the columns. The electric charges supplied to the signal lines 56 are amplified by amplifiers 62, which are connected respectively to the signal lines 56. The amplifiers 62 are connected through respective sample and hold circuits 64 to the multiplexer 66. The multiplexer 66 comprises a plurality of second switches SW2 for successively switching between the signal lines 56, and an address decoder 68 for outputting a selection signal for selecting one of the second switches SW2 at a time. The address decoder 68 is supplied with an address signal from the detector controller 42. The multiplexer 66 has an output terminal connected to an A/D converter 70. Radiographic image signals generated by the multiplexer 66 based on electric charges from the sample and hold circuits 64 are converted by the A/D converter 70 into digital image signals representing radiographic image information, which is supplied to the detector controller 42. The detector controller 42 supplies the digital image signals to the image memory 30 (see FIG. 1), which stores the supplied digital image signals. In summary, each time the first image generating apparatus 10A operates in a radiographic image capturing mode, the radiation detecting device 12 outputs a radiographic image. Radiographic images, which are successively output from the radiation detecting device 12, are stored in the image memory 30 in chronological order, for example.

The first image generating apparatus 10A also has a preview display unit 100 for displaying on the display monitor 17 preview images based on the radiographic images that are successively output from the radiation detecting device 12, for thereby assisting the operator to determine whether to carry out a tomosynthesis image capturing mode or not.

The preview display unit 100 processes radiographic images that are successively output from the radiation detecting device 12, and displays the processed radiographic images as preview images, in the following manner:
(1) The preview display unit 100 displays radiographic images at a lower resolution than the radiographic images that are successively output from the radiation detecting device 12. The preview display unit 100 displays odd-numbered or even-numbered ones of the radiographic images successively output from the radiation detecting device 12.
(2) The preview display unit 100 displays radiographic images of a particular region or a specified region (e.g., a left lung or a right lung).
(3) The preview display unit 100 displays at least every pth (p = 1, 2, 3, ···) output radiographic image. For example, the preview display unit 100 displays every fifth radiographic image, i.e., a fifth radiographic image, a tenth radiographic image, a fifteenth radiographic image, etc. The statement "every fifth radiographic image" referred to above is given by way of example only. The preview display unit 100 may display any desired radiographic images, e.g., every third radiographic image, every fourth radiographic image, every sixth radiographic image, every tenth radiographic image, etc., depending on the rate at which radiographic images are captured in the radiographic image capturing mode.

Since radiographic images of the subject 24 captured in the radiographic image capturing mode are displayed on the display monitor 17, the first image generating apparatus 10A enables the operator to easily recognize the level of body motion of the subject 24 that occurs during the radiographic image capturing process. Therefore, the first image generating apparatus 10A can operate efficiently, does not cause the subject 24 to be exposed to an excessively high dosage of radiation, and does not require the subject 24 to be constrained for an unduly long period of time.

The preview display unit 100 may display radiographic images along with a reference pattern on the display monitor 17. The reference pattern may be a crisscross pattern, whose intersection is positioned at the center of the screen of the display monitor 17. Alternatively, the reference pattern may be a grid pattern made up from a combination of a frame pattern and a crisscross pattern, for example. By viewing radiographic images displayed along with a reference pattern on the display monitor 17, the operator can easily recognize body motions of the subject 24, and thereby determine whether to carry on with the tomosynthesis image capturing mode or not.

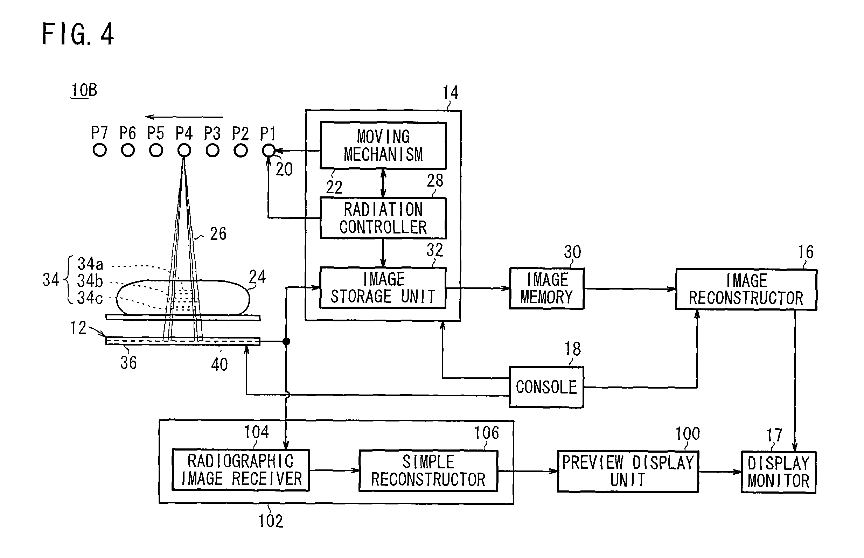

An image generating apparatus according to a second embodiment of the present invention (hereinafter referred to as a "second image generating apparatus 10B") will be described below with reference to FIGS. 4 through 8. Features of the second image generating apparatus 10B, which are identical to those of the first image generating apparatus 10A, are denoted by identical reference characters and will not be described in detail below.

As shown in FIG. 4, the second image generating apparatus 10B is similar to the first image generating apparatus 10A, but differs therefrom as follows:

The second image generating apparatus 10B includes a preview processor 102 for generating a preview tomography image based on radiographic images successively output from the radiation detecting device 12.

The preview processor 102 generates the preview tomography image based on radiographic images successively output from the radiation detecting device 12, and before the radiographic images have been stored in the image memory 30. More specifically, the preview processor 102 includes a radiographic image receiver 104 for receiving the output radiographic images, one by one, from the radiation detecting device 12, and a simple reconstructor 106 for sequentially processing the received radiographic images by way of simple backprojection in order to gradually generate a reconstructed image. The preview processor 102 displays the reconstructed image, as it is being gradually generated by the simple reconstructor 106, on the display monitor 17. In summary, the second image generating apparatus 10B sequentially processes radiographic images successively output from the radiation detecting device 12 by way of simple backprojection, and then displays the processed radiographic images on the display monitor 17.

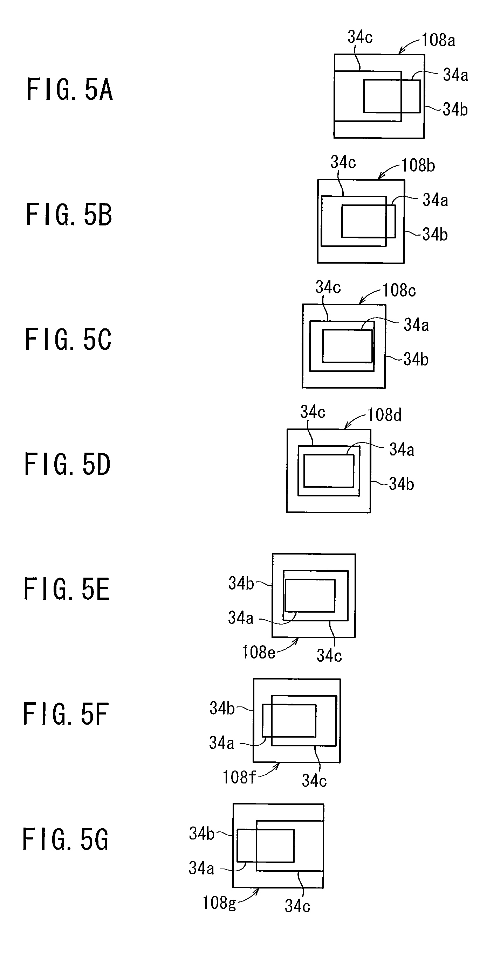

Differences between the image processing sequence of the image reconstructor 16 and the image processing sequence of the preview processor 102 will be described below with reference to FIGS. 5A through 7.

After the tomosynthesis image capturing mode has been completed, the image reconstructor 16 processes a given number of radiographic images stored in the image memory 30 in order to generate or reconstruct a diagnostic tomography image of the subject 24 who lies parallel to the detecting surface of the radiation detecting device 12. The reconstructing process used by the image reconstructor 16 to reconstruct the diagnostic tomography image may be a simple backprojection process or a filtered backprojection process, as described above.

The subject 24 may have a plurality of regions of interest, which are successively arranged in the thickness direction of the subject 24. In FIG. 4, the subject 24 has three regions of interest, i.e., a first region 34a of interest, a second region 34b of interest, and a third region 34c of interest.

For the sake of brevity, it shall be assumed that the reconstructing process used by the image reconstructor 16 to reconstruct a diagnostic tomography image is a simple backprojection process. The radiographic image acquiring assembly 14 moves the radiation applicator 20 successively to seven different positions, i.e., first through seventh positions P1 through P7, in confronting relation to the radiation detecting device 12, and controls the radiation applicator 20 to apply radiation 26 to the subject 24 who lies parallel to the radiation detecting device 12, from different directions while the radiation applicator 20 is positioned successively at the first through seventh positions P1 through P7. The radiation detecting device 12 captures seven radiographic images, i.e., first through seventh radiographic images 108a through 108g, as shown in FIGS. 5A through 5G.

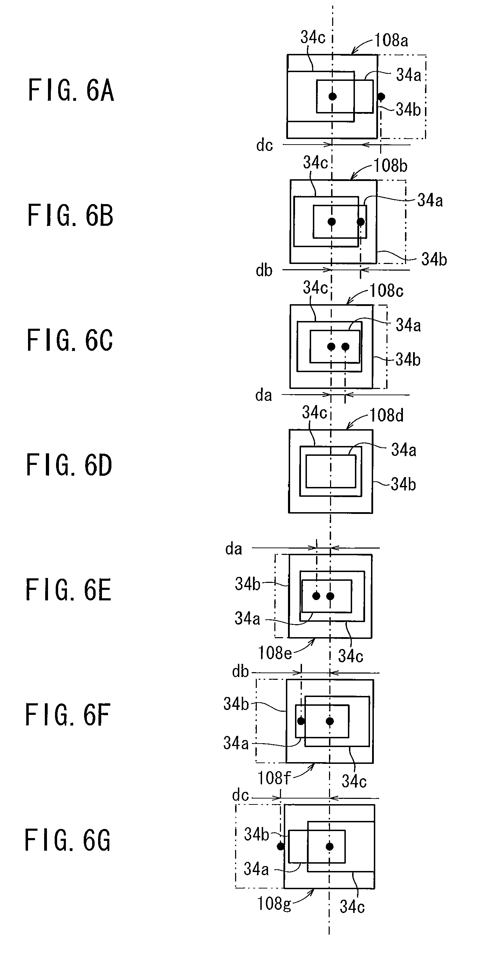

The first through third regions 34a through 34c, which are included in the first through seventh radiographic images 108a through 108g, have different image positions depending on the distances (heights) from the detecting surface of the radiation detecting device 12. In a simple backprojection process, based on the different image positions of the first through third regions 34a through 34c, the first through seventh radiographic images 108a through 108g are horizontally shifted to emphasize a certain region of interest, i.e., a certain sectional plane. If the second region 34b of interest is to be emphasized, then, as shown in FIGS. 6A through 6G, using the fourth radiographic image 108d as a reference, the third radiographic image 108c is shifted with respect to the fourth radiographic image 108d by -da, the fifth radiographic image 108e by +da, the second radiographic image 108b by -db (|db| > |da|), the sixth radiographic image 108f by +db, the first radiographic image 108a by -dc (|dc| > |db|), and the seventh radiographic image 108g by +dc. The first through seventh radiographic images 108a through 108g, except for the fourth radiographic image 108d, are added together into a tomography image (diagnostic tomography image), which represents, with emphasis, the second region 34b of interest. Diagnostic images, which represent and emphasize the first and third regions 34a, 34c of interest, can similarly be generated.

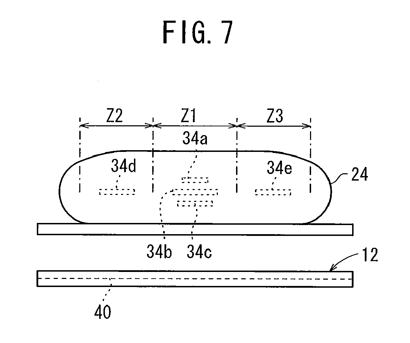

The subject 24 may have a plurality of regions of interest located within a plurality of zones. In FIG. 7, the subject 24 has three regions of interest in a first zone Z1 (i.e., a first region 34a of interest, a second region 34b of interest, and a third region 34c of interest), a single region of interest in a second zone Z2 (i.e., a fourth region 34d of interest), and a single region of interest in a third zone Z3 (i.e., a fifth region 34e of interest). For emphasizing a certain region of interest selected from among the first through fifth regions 34a through 34e of interest, the first through seventh radiographic images 108a through 108g also are shifted and added together.

The preview processor 102 generates a preview tomography image for use in confirming body motions of the subject 24 during the tomosynthesis image capturing mode. The preview processor 102 performs its image processing sequence faster than the image reconstructor 16 performs its processing sequence. According to the present embodiment, each time the radiographic image capturing mode is carried out, the radiographic image receiver 104 receives a radiographic image from the radiation detecting device 12, whereupon the simple reconstructor 106 processes the received radiographic image by way of simple backprojection. The preview display unit 100 then displays the processed radiographic image on the display monitor 17. Accordingly, the display monitor 17 displays a preview tomography image while the tomography image of a single region of interest is being generated. The single region of interest may be the second region 34b of interest.

The preview processor 102 is capable of performing its image processing sequence faster than the image reconstructor 16 performs its processing sequence, in light of the following facts:
(1) The image reconstructor 16 carries out a filtered backprojection process, whereas the preview processor 102 carries out a simple backprojection process.
(2) Whereas the image reconstructor 16 generates tomography images with respect to a plurality of regions of interest for one zone, the preview processor 102 generates a tomography image only with respect to a single region of interest.
(3) The preview processor 102 generates tomography images at a lower resolution than that of the tomography images generated by the image reconstructor 16. For example, the preview processor 102 uses odd-numbered or even-numbered pixel data of the received radiographic images, and processes the odd-numbered or even-numbered pixel data by way of simple backprojection in order to generate a preview tomography image.
(4) The preview processor 102 generates or reconstructs a preview tomography image by processing fewer radiographic images than the radiographic images used by the image reconstructor 16. For example, the preview processor 102 processes every qth (q = 1, 2, 3, ···) radiographic image output from the radiation detecting device 12, e.g., every fifth radiographic image, i.e., a fifth radiographic image, a tenth radiographic image, a fifteenth radiographic image, etc., by way of simple backprojection, in order to update the preview tomography image. The preview display unit 100 updates the displayed image each time the preview tomography image is updated. A first radiographic image may be included within the radiographic images processed by the preview processor 102. The phrase "every fifth radiographic image" referred to above is given by way of example only. The preview processor 102 may process any desired radiographic images, e.g., every third radiographic image, every fourth radiographic image, every sixth radiographic image, every tenth radiographic image, etc., depending on the rate at which radiographic images are captured in the radiographic image capturing mode. Alternatively, the preview processor 102 may receive a radiographic image produced in an odd-numbered radiographic image capturing mode (i.e., a radiographic image capturing mode carried out at an odd-numbered position in confronting relation to the radiation detecting device 12) or an even-numbered radiographic image capturing mode (i.e., a radiographic image capturing mode carried out at an even-numbered position in confronting relation to the radiation detecting device 12), and may process the received radiographic image by way of simple backprojection in order to produce a preview tomography image.
(5) The image reconstructor 16 generates a diagnostic tomography image over a plurality of zones (e.g., first through third zones Z1 through Z3 as shown in FIG. 7), whereas the preview processor 102 generates a preview tomography image with respect to a single particular zone or a single specified zone (e.g., the first zone Z1). In the illustrated embodiment, the preview processor 102 generates a preview tomography image with respect to the first region 34a of interest in the first zone Z1 However, the preview processor 102 may generate a preview tomography image with respect to the second region 34b of interest in the first zone Z1, the fourth region 34d of interest in the second zone Z2, or the fifth region 34e of interest in the third zone Z3.

If the body motion of the subject 24 is small, then since the operator can recognize the tomography image, which is gradually generated when radiographic images output from the radiation detecting device 12 are successively processed by way of simple backprojection, the operator can easily judge that the tomosynthesis image capturing mode should be carried on. Conversely, if the body motion of the subject 24 is large, then since a tomography image is not displayed, even when radiographic images output from the radiation detecting device 12 are successively processed by way of simple backprojection, the operator can easily judge that the tomosynthesis image capturing mode should be called off. If the operator judges that the tomosynthesis image capturing mode should be called off, then the tomosynthesis image capturing mode is immediately suspended, and preparations are initiated in order to recapture radiographic images of the subject 24, for example.

According to the related art, even when the level of body motion of the subject is large enough to adversely affect reconstruction of a tomography image, such movement is not detected, and the tomosynthesis image capturing mode is continued. Therefore, the tomosynthesis image capturing mode as a whole is wasted, and another image capturing process will be required. Consequently, the radiographic tomography image generating apparatus is potentially low in efficiency, may cause the subject to be exposed to an excessively high dosage of radiation, and may require the subject to be constrained for an unduly long period of time.

The second image generating apparatus 10B according to the present embodiment displays, on the display monitor 17, a tomography image, which is generated by the preview processor 102 during the tomosynthesis image capturing mode. Accordingly, the operator can easily recognize the level of body motion of the subject 24, and can easily determine whether or not to call off the tomosynthesis image capturing mode. If the operator decides that the tomosynthesis image capturing mode should be called off, then subsequent radiographic image capturing processes will not be wasted, and preparations can quickly be initiated in order to recapture radiographic images of the subject 24. Therefore, the second image generating apparatus 10B operates efficiently, does not cause the subject 24 to be exposed to an excessively high dosage of radiation, and does not require the subject 24 to be constrained for an unduly long period of time.

In the second image generating apparatus 10B, the preview display unit 100 may also display on the display monitor 17 radiographic images processed by way of simple backprojection, along with a reference pattern. The reference pattern may be a crisscross pattern the intersection of which is positioned at the center of the screen of the display monitor 17, or a grid pattern made up of a combination of a frame pattern and a crisscross pattern, for example. By viewing radiographic images processed by way of simple backprojection and displayed along with a reference pattern on the display monitor 17, the operator can easily recognize body motions of the subject 24 and can determine whether or not to carry on with the tomosynthesis image capturing mode.

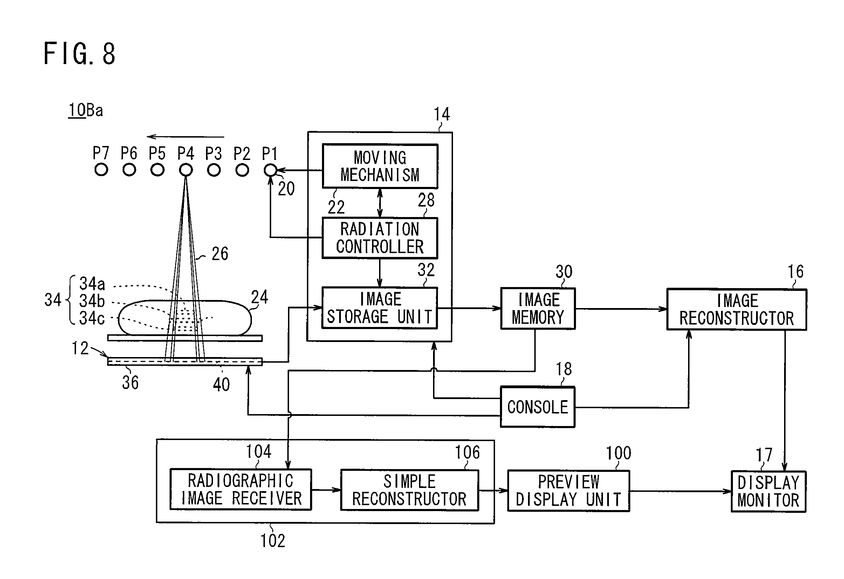

In the above second embodiment, the preview processor 102 generates a preview tomography image based on radiographic images successively output from the radiation detecting device 12, before the radiographic images have been stored in the image memory 30. FIG. 8 shows, partially in block form, a second image generating apparatus 10Ba according to a modification of the present invention. With the second image generating apparatus 10Ba, the preview processor 102 generates a preview tomography image based on radiographic images that have been stored in the image memory 30. More specifically, the preview processor 102 includes a radiographic image reader 110 for reading latest radiographic images, one by one, from the image memory 30, and a simple reconstructor 106 for sequentially processing the read radiographic images by way of simple backprojection, so as to gradually generate a reconstructed image. The preview display unit 100 displays a reconstructed image, which is gradually generated by the simple reconstructor 106, on the display monitor 17.

The modified second image generating apparatus 10Ba thus enables the operator to easily recognize the level of body motion of the subject 24. Therefore, the modified second image generating apparatus 10Ba can operate efficiently, does not cause the subject 24 to be exposed to an excessively high dosage of radiation, and does not require the subject 24 to be constrained for an unduly long period of time.

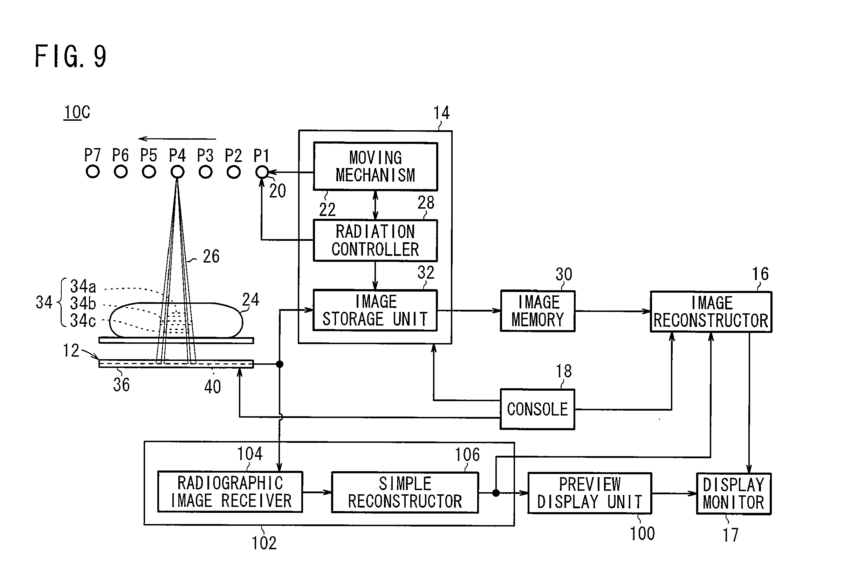

An image generating apparatus according to a third embodiment of the present invention (hereinafter referred to as a "third image generating apparatus 10C") will be described below with reference to FIGS. 9 through 11B. Features of the third image generating apparatus 10C, which are identical to those of the first and second image generating apparatus 10A, 10B, 10Ba, are denoted by identical reference characters and will not be described in detail below.

As shown in FIG. 9, the third image generating apparatus 10C is similar to the second image generating apparatus 10B, but differs therefrom as follows:

The image reconstructor 16 processes a portion of the plural radiographic images that are stored in the image memory 30 by way of backprojection, adds a reconstructed preview tomography image from the simple reconstructor 106 of the preview processor 102 in order to generate a diagnostic tomography image, and displays the generated diagnostic tomography image on the display monitor 17.

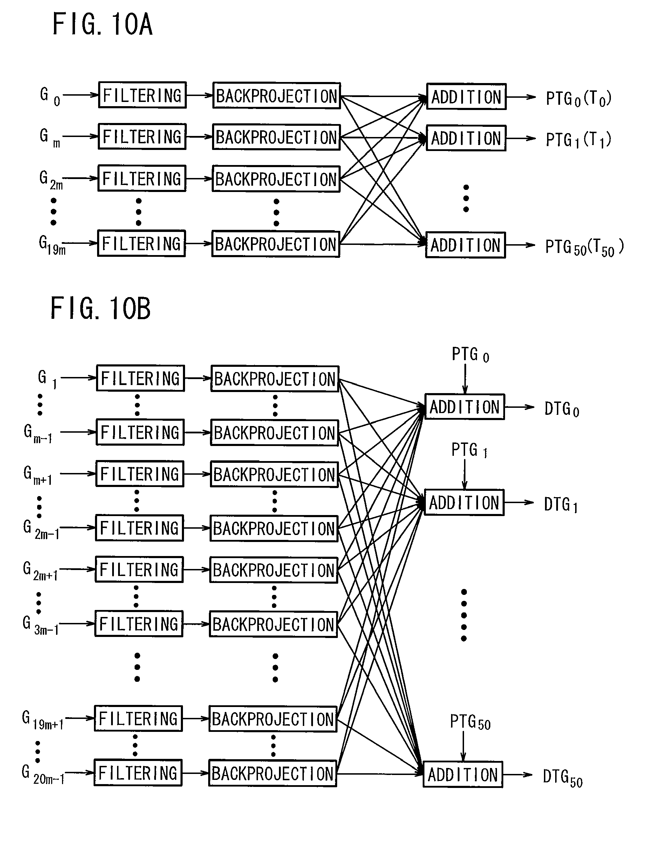

Two processing methods (a first processing method and a second processing method) for producing diagnostic tomography images will be described below with reference to FIGS. 10A through 11B.

The first processing method is a method for handling cases in which the number of diagnostic tomography images to be generated and the number of preview tomography images are the same as each other, and the number of radiographic images (captured images) for generating diagnostic tomography images and the number of radiographic images (captured images) for generating preview tomography images are different from each other.

The first processing method will be described below with reference to FIGS. 10A and 10B.

In the reconstructing process for reconstructing preview tomography images, which is carried out by the simple reconstructor 106 of the preview processor 102, every mth radiographic image is sequentially processed in order to generate preview tomography images. In this case, a preview tomography image, which is generated each time the number of radiographic images increases, may be displayed, so as to update the presently displayed preview tomography image on the display monitor 17 through the preview display unit 100.

For example, assume that 51 preview tomography images representative of respective sectional planes, which are produced by slicing a range having a thickness of 5 cm at intervals of 1 mm, are to be generated. As shown in FIG. 10A, every mth (e.g., m = 3) radiographic image, i.e., 20 radiographic images Go, Gₘ, G₂ₘ, G₃ₘ, ···, G₁₉ₘ, out of 60 radiographic images, for example, are processed by a reconstruction filter (ramp, etc.), which comprises a convolution filter, and then are processed by way of backprojection. Then, the processed radiographic images are shifted for the respective sectional planes T₀, T₁, T₂, ···, T₅₀ and added together in order to generate 51 preview tomography images PTG₀, PTG₁, PTG₂ , ··· , PTG₅₀.

In the reconstructing process for reconstructing diagnostic tomography images, which is carried out by the image reconstructor 16, the generated preview tomography images and the radiographic images that were not used in the process for reconstructing preview tomography images are used in order to generate diagnostic tomography images.

For example, assume that 51 diagnostic tomography images representative of respective sectional planes, which are produced by slicing a range having a thickness of 5 cm at intervals of 1 mm, are to be generated. As shown in FIG. 10B, radiographic images except every mth (e.g., m = 3) radiographic image, i.e., 40 radiographic images (G₁ ··· Gₘ₋₁), (Gₘ₊₁ ··· G₂ₘ₋₁), (G₂ₘ₊₁ ··· G₃ₘ₋₁), ···, (G₁₉ₘ₊₁ ··· G₂₀ₘ₋₁), out of 60 radiographic images, for example, are processed by a reconstruction filter (ramp, etc.), which comprises a convolution filter, and then are processed by way of backprojection. Then, the processed radiographic images are shifted for the respective sectional planes T₀, T₁, T₂, ··· , T₅₀ and added, and the preview tomography images corresponding to the sectional planes are added thereto in order to generate 51 diagnostic tomography images DTG₀, DTG₁, DTG₂ , ···, DTG₅₀.

The second processing method is a method for handling cases in which the number of diagnostic tomography images to be generated and the number of preview tomography images are different from each other, and the number of radiographic images (captured images) for generating diagnostic tomography images and the number of radiographic images (captured images) for generating preview tomography images are different from each other.

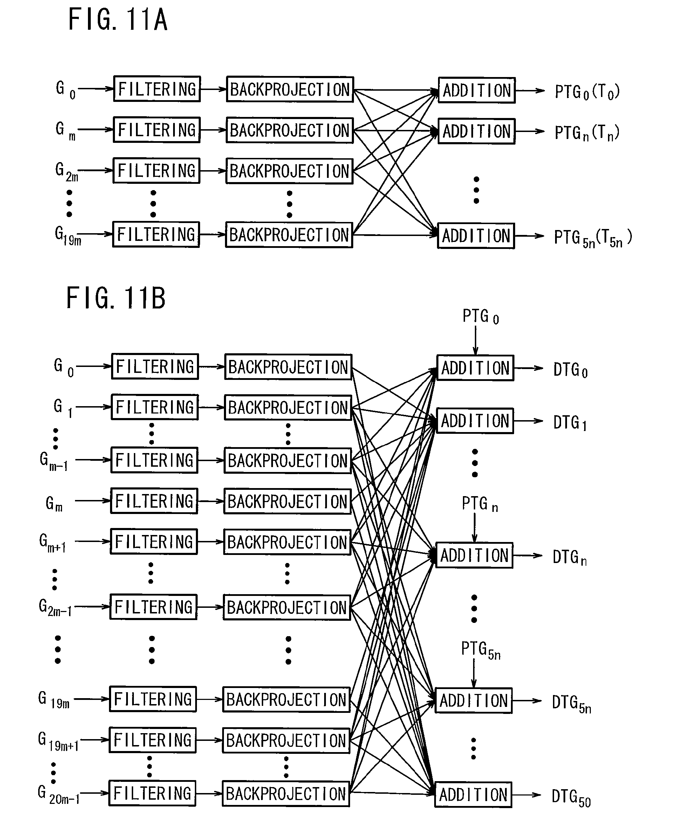

The second processing method will be described below with reference to FIGS. 11A and 11B.

In the reconstructing process for reconstructing preview tomography images, which is carried out by the simple reconstructor 106 of the preview processor 102, every mth radiographic image is sequentially processed in order to generate every nth preview tomography image. In this case, a preview tomography image, which is generated each time that the number of radiographic images increases, may be displayed, so as to update the presently displayed preview tomography image on the display monitor 17 through the preview display unit 100.

For example, assume that 6 preview tomography images representative of respective sectional planes are to be generated, which are produced by slicing a range having a thickness of 5 cm at intervals of 1 mm. As shown in FIG. 11A, every mth (e.g., m = 3) radiographic image, i.e., 20 radiographic images Go, Gₘ, G₂ₘ, ···, G₁₉ₘ, out of 60 radiographic images, for example, are processed by a reconstruction filter (ramp, etc.), which comprises a convolution filter, and then are processed by way of backprojection. Then, the processed radiographic images are shifted for every nth (e.g., n = 10) sectional planes T₀, Tₙ, T₂ₙ, ···, T₅ₙ and are added in order to generate 6 preview tomography images PTG₀, PTGₙ, ···, PTG₅ₙ.

In the reconstructing process for reconstructing the diagnostic tomography images, which is carried out by the image reconstructor 16, the generated preview tomography images and the radiographic images that were not used in the process of reconstructing the preview tomography images are used together in order to generate diagnostic tomography images. For sectional planes for which no preview tomography images are generated, radiographic images are sequentially processed in order to generate diagnostic tomography images.

For example, assume that 51 diagnostic tomography images representative of respective sectional planes are to be generated, which are produced by slicing a range having a thickness of 5 cm at intervals of 1 mm. As shown in FIG. 11B, radiographic images except every mth (e.g., m = 3) radiographic image, i.e., 40 radiographic images (G₁ ··· Gₘ₋₁), (Gₘ₊₁ ··· G₂ₘ₋₁), ···, (G₁₉ₘ₊₁ ··· G₂₀ₘ₋₁), out of 60 radiographic images, for example, are processed by a reconstruction filter (ramp, etc.), which comprises a convolution filter, and then are processed by way of backprojection. Then, the processed radiographic images are shifted for the respective sectional planes T₀, Tₙ, T₂ₙ, ···, T₅ₙ and added, and preview tomography images PTG₀, PTGₙ, PTG₂ₙ, ···, PTG₅ₙ corresponding to the sectional planes T₀, Tₙ, T₂ₙ, ···, T₅ₙ are added thereto in order to generate diagnostic tomography images DTG₀, DTGₙ, DTG₂ₙ, ···, DTG₅ₙ.

For sectional planes for which no preview tomography images have been generated, 60 radiographic images, for example, are processed by a reconstruction filter (ramp, etc.), which comprises a convolution filter, and then are processed by way of backprojection. Then, the processed radiographic images are shifted for the respective sectional planes and added in order to generate diagnostic tomography images (DTG₁, ···, DTGₙ₋₁), (DTGₙ₊₁, ···, DTG₂ₙ₋₁), ···, DTG₅₀, which are representative of the sectional planes (T₁, ···, Tₙ₋₁), (Tₙ₊₁, ···, T₂ₙ₋₁), ···, T₅₀.

In this manner, 51 diagnostic tomography images DTG₀, DTG₁, DTG₂, ···, DTG₅₀ are generated.

The third image generating apparatus 10C offers the same advantages as the second image generating apparatus 10B. In addition, since the results of the process for reconstructing preview tomography images, which is carried out by the simple reconstructor 106 of the preview processor 102, are utilized in the process of reconstructing the diagnostic tomography images, which is carried out by the image reconstructor 16, the third image generating apparatus 10C is capable of increasing the efficiency with which the image reconstructing process is performed.

In the reconstructing process for reconstructing preview tomography images with the third image generating apparatus 10C, radiographic images are processed by means of a reconstruction filter, and then are processed by way of backprojection. However, the radiographic images may directly be processed by way of backprojection. In the reconstructing process for reconstructing diagnostic tomography images, the radiographic images also are processed by means of a reconstruction filter, and then are processed by way of backprojection. However, the radiographic images also may be directly processed by way of backprojection.

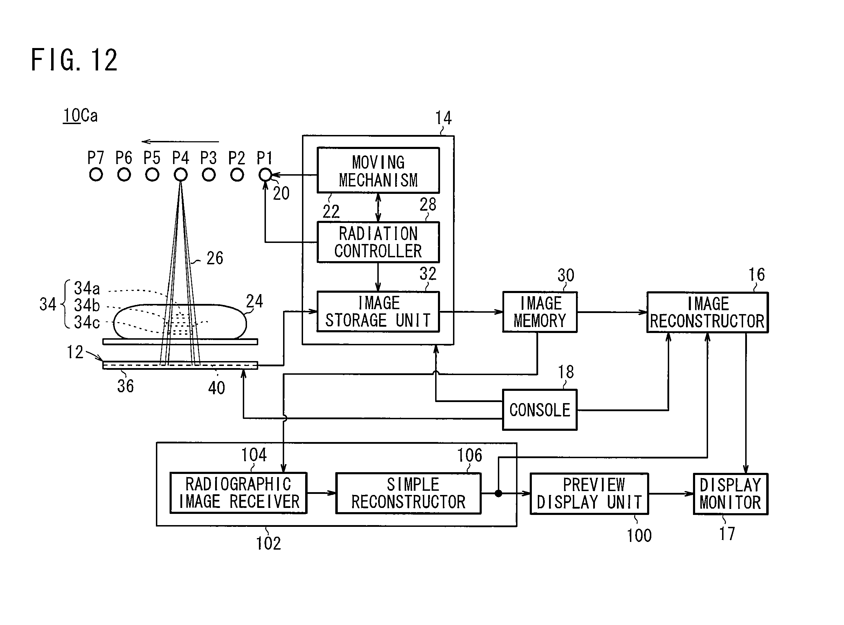

In the third image generating apparatus 10C, the preview processor 102 generates a preview tomography image based on radiographic images successively output from the radiation detecting device 12, and prior to the radiographic images being stored in the image memory 30. FIG. 12 shows, partially in block form, a third image generating apparatus 10Ca according to a modification of the present invention. With the modified third image generating apparatus 10Ca, the preview processor 102 generates a preview tomography image based on radiographic images, which have been stored in the image memory 30. The modified third image generating apparatus 10Ca offers the same advantages as the modified second image generating apparatus 10Ba. In addition, since the results of the process of reconstructing preview tomography images, which is carried out by the simple reconstructor 106 of the preview processor 102, can be utilized during the process of reconstructing diagnostic tomography images, which is carried out by the image reconstructor 16, the modified third image generating apparatus 10Ca is capable of increasing the efficiency at which the image reconstructing process is preformed.

The present invention is not limited to the various embodiments described above, but various changes and modifications may be made to the embodiments within the scope of the invention.

For example, in the illustrated radiographic tomography image generating apparatus, the radiation detector 40 of the radiation detecting device 12 is of a direct conversion type, which directly converts the dosage of applied radiation 26 directly into electric signals using the photoelectric conversion layer 51. However, the radiographic tomography image generating apparatus may also employ a radiation detector of a direct conversion type, which includes a scintillator for converting applied radiation 26 into visible light, together with a solid-state detecting device made of amorphous silicon (a-Si) or the like for converting the visible light into electric signals (see, e.g., Japanese Patent No. 3494683).

Alternatively, the radiographic tomography image generating apparatus may employ a light-conversion radiation detector for acquiring radiographic image information. Such a light-conversion radiation detector operates as follows: When radiation is applied to a matrix of solid-state detecting devices, the solid-state detecting devices store an electrostatic latent image depending on the dosage of applied radiation. For reading the stored electrostatic latent image, reading light is applied to the solid-state detecting devices in order to cause the solid-state detecting devices to generate an electric current, which represents the radiation image information. When erasing light is applied to the radiation detector, radiographic image information representing a residual electrostatic latent image is erased from the radiation detector, thus enabling the radiation detector to be reused (see Japanese Laid-Open Patent Publication No. 2000-105297).

While the illustrated radiation detector 40 employs TFTs 52, alternatively, the radiation detector may employ any of various other image capturing devices, such as a CMOS (Complementary Metal-Oxide Semiconductor) image sensor or a CCD (Charge-Coupled Device) image sensor, wherein electric charges are shifted and transferred by shift pulses corresponding to gate signals used in the TFTs 52.

## Claims

1. A radiographic tomography image generating apparatus comprising:
a radiation detecting device (12);
a radiation applicator (20) disposed in confronting relation to the radiation detecting device (12);
a radiographic image acquiring assembly (14) for moving the radiation applicator (20) successively to a plurality of positions, controlling the radiation applicator (20) to apply radiation (26) to a subject (24) disposed over the radiation detecting device (12) in different directions while the radiation applicator (20) moves successively to the positions, and storing a plurality of radiographic images output successively from the radiation detecting device (12) in an image memory (30);
an image reconstructor (16) for processing the radiographic images stored in the image memory (30) to reconstruct a diagnostic tomography image of the subject (24) according to a reconstructing process; and
a preview display unit (100) for displaying preview images based on the radiographic images output successively from the radiation detecting device (12).

2. A radiographic tomography image generating apparatus according to claim 1, wherein the preview display unit (100) displays the preview images by displaying radiographic images at a lower resolution than the radiographic images that are output successively from the radiation detecting device (12).

3. A radiographic tomography image generating apparatus according to claim 1, wherein the preview display unit (100) displays the preview images by displaying radiographic images of a particular region or a specified region.

4. A radiographic tomography image generating apparatus according to claim 1, wherein the preview display unit (100) displays the preview images by sequentially displaying at least every pth (p = 1, 2, 3, ···) output radiographic image.

5. A radiographic tomography image generating apparatus according to claim 1, wherein the preview display unit (100) displays the preview images by displaying radiographic images along with a reference pattern.

6. A radiographic tomography image generating apparatus according to claim 1, further comprising:
a preview processor (102) for generating a preview tomography image by processing radiographic images that are output successively from the radiation detecting device (12), according to a simpler reconstructing process than the reconstructing process carried out by the image reconstructor (16),
wherein the preview display unit (100) displays the preview tomography image.

7. A radiographic tomography image generating apparatus according to claim 6, wherein the preview processor (102) generates the preview tomography image at a lower resolution than the diagnostic tomography image reconstructed by the image reconstructor (16).

8. A radiographic tomography image generating apparatus according to claim 6, wherein the image reconstructor (16) generates the diagnostic tomography image, which represents a plurality of regions in the subject (24), and the preview processor (102) generates the preview tomography image, which represents a particular region or a specified region among the plurality of regions.

9. A radiographic tomography image generating apparatus according to claim 6, wherein the preview processor (102) updates the preview tomography image by processing at least every qth (q = 1, 2, 3, ···) radiographic image output from the radiation detecting device (12) by way of backprojection, and the preview display unit (100) updates the displayed preview tomography image each time the preview processor (102) updates the preview tomography image.

10. A radiographic tomography image generating apparatus according to claim 6, wherein the preview processor (102) controls the preview display unit (100) to display the preview tomography image along with a reference pattern.

11. A radiographic tomography image generating apparatus according to claim 6, wherein the image reconstructor (16) generates diagnostic tomography images, which represent at least two sectional planes in the subject (24), and the preview processor (102) generates the preview tomography image, which represents a particular one of the at least two sectional planes.

12. A radiographic tomography image generating apparatus according to claim 6, wherein the image reconstructor (16) generates the diagnostic tomography image by way of filtered backprojection, and the preview processor (102) generates the preview tomography image by way of simple backprojection.

13. A radiographic tomography image generating apparatus according to claim 6, wherein the image reconstructor (16) generates the diagnostic tomography image while also using the preview tomography image generated by the preview processor (102).

14. A radiographic tomography image generating apparatus according to claim 13, wherein the preview processor (102) generates the preview tomography image by processing every mth (m = 1, 2, 3, ···) radiographic image of the radiographic images that are output successively from the radiation detecting device (12), and the image reconstructor (16) generates the diagnostic tomography image using the generated preview tomography image and radiographic images that are not used by the preview processor (102).

15. A radiographic tomography image generating apparatus according to claim 13, wherein the preview processor (102) generates every nth (n = 1, 2, 3, ···) preview tomography image by processing every mth (m = 1, 2, 3, ···) radiographic image of the radiographic images that are output successively from the radiation detecting device (12), and the image reconstructor (16) generates the diagnostic tomography image using the generated preview tomography images and radiographic images that are not used by the preview processor (102) with respect to sectional planes in the subject (24) for which the diagnostic tomography image is generated, and the image reconstructor (16) generates the diagnostic tomography image using radiographic images with respect to sectional planes in the subject (24) for which the diagnostic tomography image is not generated.

16. A radiographic tomography image generating apparatus according to any of claims 6 to 15, wherein the preview processor (102) generates the preview tomography image based on the radiographic images stored in the image memory (30).

17. A radiographic tomography image generating apparatus according to any of claims 6 to 15, wherein the preview processor (102) generates the preview tomography image based on radiographic images that are output from the radiation detecting device (12), before the radiographic images are stored in the image memory (30).
